# EUROPEAN PATENT APPLICATION

(11) **EP 1 674 085 A1**
(43) Date of publication of application: **28.06.2006**
(21) Application number: 04447294.2
(22) Date of filing: 22.12.2004
(51) Int. Cl.: A61K 9/51, A61K 9/16

(54) **Solid lipidic particles as pharmaceutically acceptable fillers or carriers for inhalation**

(71) Applicant: UNIVERSITE LIBRE DE BRUXELLES, 1050 Bruxelles (BE)
(72) Inventor: Amighi, Karim, 3080 Tervuren (BE); Sebti, Thami, B-1040 Brussels (BE)
(74) Representative: Van Malderen, Joëlle

(57) **Abstract**

The present invention relates to new compositions of (active) solid lipidic particles (SLP), e.g. for inhalation, and their use as carriers or as fillers in pharmaceutical compositions.

It also relates new formulations obtained by mixing a SLP composition of the invention and a (micronized) active compound.

It further relates to a method for fabricating said compositions of (active) solid lipidic particles.

## Description

### Field of the invention

The present invention relates to new compositions of (active) solid lipidic particles (SLP), e.g. for inhalation, and their use as carriers or as fillers in pharmaceutical compositions.

It also relates to new formulations obtained by mixing a SLP composition of the invention and a micronized active compound.

It further relates to a method for fabricating said compositions of (active) solid lipidic particles.

### Background of the invention

It is known to administer to patients drugs in the form of fine active particles.

The pulmonary route may present several advantages in the treatment of some diseases, in particular in the treatment of respiratory diseases, over the administration of the same drugs by other routes leading to the systemic delivery of such drugs.

Drug inhalation enables a rapid and predictable onset of action and induces fewer side effects than does administration by other routes.

However, these advantages are often associated to a limited deposition of the inhaled dose and a short duration action because of the protective mechanisms of the lungs (mucociliary clearance, expectoration, enzymatic system, etc) .

In other respects, the respiratory tract possesses specific characteristics, such as an exceedingly large surface area (up to 140 m²), a thin absorption mucosal membrane (0,1 - 0,2 µm) and lacks of first-pass hepatic metabolism, which makes it very attractive as a systemic administration route.

Three main delivery systems have been devised for the inhalation of aerosolized drug, namely, pressurized metered-dose inhalers (MDIs), nebulisers and dry powder inhalers (DPIs).

The latter are today the most convenient alternative to MDIs as they are breath-actuated and do not require the use of any propellants.

The deposition site and the efficiency of inhaled aerosols in the respiratory tract are critically influenced by the dispersion properties of the particles, and the aerodynamic diameter, size distribution, shape and density of generated particles.

For an effective inhalation therapy, inhaled active particles should have an aerodynamic diameter between about 0,5 and 5 µm to reach the lower airways.

Since micronized drug particles are generally very cohesive and characterized by poor flowing properties, they are usually blended, in dry powder formulations, with coarse and fine carrier particles. These carrier particles are generally carbohydrates, mainly mannitol and lactose, which are approved by the Food and Drug Administration (FDA).

Furthermore, the carrier particles should be chemically and physically stable, inert to the drug substance and should not exhibit harmful effects, especially on the respiratory tract.

In fact, the number of carriers or fillers acceptable for inhalation purpose is very limited because of many different requirements to meet before they are used.

WO02/43693 discloses compositions for inhalation comprising active particles, cholesterol particles and particles of excipient material, i.e. particles of carrier.

There is still a need for carriers or fillers that are able to overcome the problems related with the pulmonary administration of drugs such as the limited drug deposition, the irritation of upper airways, the rapid elimination of inhaled particles, the short duration action, etc.

This invention proposes the possibility to obtain different compositions for pulmonary administration having satisfactory properties in term of increasing drug deposition and/or delaying or accelerating drug release rate.

### Summary of the invention

The present invention provides a new composition of solid lipidic particles (a SLP composition), each particle comprising biocompatible phospholipids and at least one additional biocompatible lipidic compound.

The present invention also provides new formulations using solid lipidic particles (SLPs) of the invention, as pharmaceutically acceptable carriers.

A SLP composition of the invention can be used as carrier, with micronized active compounds in order to promote the release of the active particles from the carrier particles on the actuation of the inhaler, improving the drug deposition.

A method for preparing a SLP composition according to the invention comprises the steps of (a) preparing a solution or a suspension containing said biocompatible phospholipids and said other biocompatible lipidic compound(s), and (b) spray-drying said solution or suspension.

Another object of the present invention relates to a composition (an active SLP composition) consisting of solid particles, each particle comprising biocompatible phospholipids, at least one other biocompatible lipidic compound and at least one active compound.

A method for preparing said active SLP composition according to the invention comprises the steps of (a) preparing a solution or a suspension containing said biocompatible phospholipids, said other biocompatible lipidic compound(s), and said active compound(s), and (b) spray-drying said solution or suspension.

Advantageously, the additional biocompatible lipidic compound is selected from the group consisting of glycerol esters (e.g. mono-, di-, and tri-glycerides, in particular, glycerol monostearate, glycerol behenate), fatty alcohols (preferably cetyl alcohol, steary alcohol cetostearyl alcohol or fatty alcohols with more than 18 carbon atoms), fatty acids (preferably palmitic acid, or fatty acids with more carbon atoms such as stearic acid, behenic acid, etc.), ethers of fatty alcohols, esters of fatty acids, hydrogenated oils, polyoxyethylenated derivatives, sterols (e.g. cholesterol, cholesterol esters), and any derivatives thereof.

Advantageously, the additional biocompatible lipidic compound is a solid material at ambient temperature.

Preferably, the biocompatible phospholipids and the additional biocompatible lipidic compounds of a composition of the invention are characterized by a high phase transition temperature (T_{c}), preferably by a T_{c} higher than about 35°C, more preferably higher than about 45°C, and even more preferably higher than about 50°C.

Advantageously, a composition of the invention consists of particles having a mean size between about 0,2 µm and 200 µm, preferably in the range of about 0,2 µm to about 80 µm, and more preferably in the range of about 0,5 µm to about 20 µm.

### Brief description of the drawings

Figure 1 shows the structural formula of phospholipids that can be used in a composition of the invention.

Figure 2 shows a modified commercially available spray dryer. Some modifications have been made in order to improve the drying efficiency and the product yield obtained by spray drying the solutions or suspensions containing lipidic compounds.

Figure 3 shows the deposition pattern of the formulations given in example 1. The best deposition pattern with the highest FPD values was obtained for SLPs formulation containing the cholesterol / phospholipids weight ratio of 90/10.

Figure 4 shows SEM (scanning electron microscope) microphotographs, at different magnifications of bulk Phospholipon 90H, cholesterol and budesonide powders, and spray dried SLP composition(as lipidic carrier) and a 2% budesonide physical blend formulation.

The SLPs show spherical structures consisting of many tiny spherical particles, approximately 0.25 - 2 µm in diameter slightly fused and agglomerated. In the physical blends, aggregates of flat and irregularly shaped particles of budesonide surround and interact with the spherical SLPs.

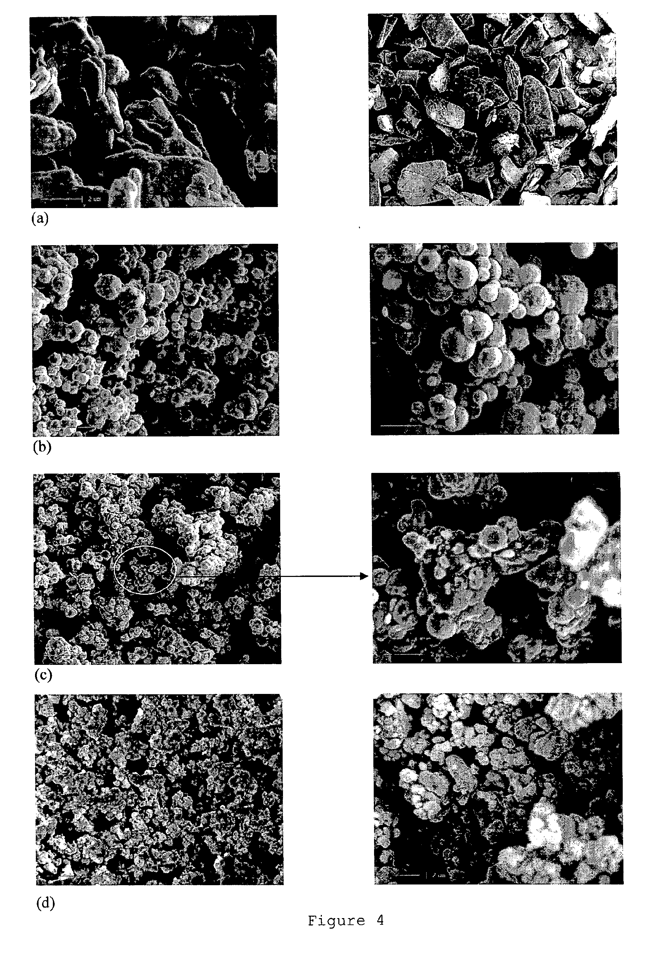

This figure 4 shows SEM microphotographs (at different magnifications) of : (a) bulk Phospholipon 90H powder (left) and cholesterol (right) used to prepare the solutions for spray-drying, (b) the spray-dried SLPs (lipid carrier), (c) the SLP(90%C10%P) + 2%Bud. physical blend formulation, and (d) budesonide (raw material).

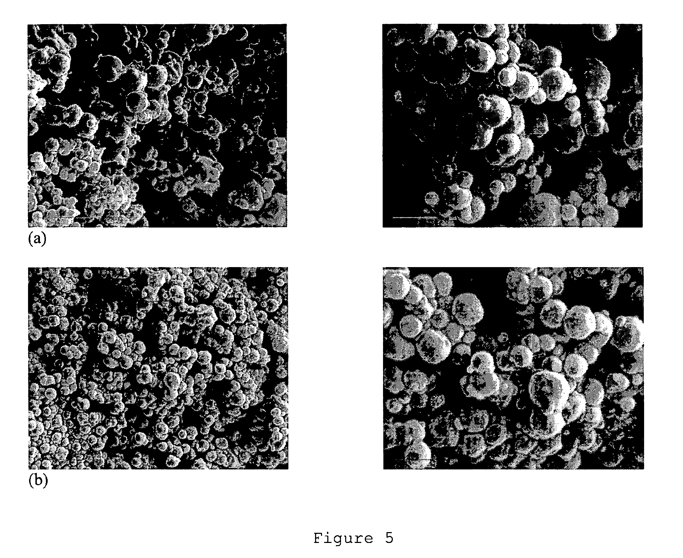

Figure 5 shows that the size and the morphological characteristics of matrix active SLPs are similar to that of the SLPs (lipidic carrier). It shows SEM micrographs (at different magnifications) of: (a) the spray-dried lipidic excipients, (b) the 90%C08%P02%B lipid matrix formulation.

### Description of the invention

A composition according to the present invention consists of solid particles, each particle comprising biocompatible phospholipids, at least one additional biocompatible lipidic compound and optionally at least one active compound.

In particular, said additional biocompatible lipidic compound(s) is/are not (a) phospholipid(s).

The term "compound" is also referred to herein as ingredient, agent or substance.

A composition of the invention can refer to a "SLP composition", to an "active SLP composition" according to the invention, and/or to a composition comprising (active) SLPs and at least one active compound in form of particles, the latter composition can also be referred to as "formulation".

The term "SLP" or "SLPs" in the context of the present invention refers to solid lipidic particles, each particle comprising or consisting (essentially) of biocompatible phospholipids and at least one additional biocompatible lipidic compound.

The term "active SLP" or "active SLPs" in the context of the present invention refers to SLPs wherein each particle further comprises at least one active compound.

The terms "biocompatible phospholipid(s)" and "biocompatible lipidic compound(s)" in the context of the present invention refer to respectively phospholipid(s) and lipidic compound(s), natural or synthetic, that are known to be biologically compatible, i.e. that should not produce any toxic, injurious or immunologically harmful response in living tissue.

A SLP composition of the invention can be used as a carrier, i.e. it can be mixed with an active compound, for improving the drug lung deposition of said active compound.

An active SLP composition of the invention can be formulated as lipidic matrices or as lipid-coated active ingredient for entrapping both water-soluble and water-insoluble drugs in order to avoid a rapid drug release and absorption, especially when the proportion of the additional biocompatible lipid in the composition is high. In this case, the characteristic peak effect and the limited duration of action generally associated with the pulmonary administration of drugs can be respectively attenuated and improved.

An active SLP composition of the invention can also be formulated as lipidic matrices or as lipid-coated active ingredient for entrapping water-insoluble drugs in order to promote the drug release and absorption, when the proportion of phospholipids in the composition is increased.

Moreover, as a dried material they (SLPs and active SLPs) offer a better stability (protection of drug in the hydrophobic environment) and a higher encapsulation efficiency (than liposomes for example).

Contrary to the classical hydrophilic excipients generally used in DPIs (carbohydrates), the hydrophobic nature of the SLPs associated with the active compound permits to reduce the absorption of the ubiquitous vapour leading to a reduction of the aggregation and the adhesion of particles.

This improves the flowing property of the particles into the inhalation device, in particular during filling process, ensures accurate dosing of active ingredients and increases the dispersing property of cohesive dry particles during emission.

In an active SLP composition of the present invention, each SLP further comprises an active compound (or active ingredient). Said active compound is thus embedded in physiological lipids for a better tolerance in the pulmonary tract, reducing the inherent local irritation generally associated with DPIs.

An active SLP composition of the invention allows protection of the pulmonary tract against irritating drugs and excipients.

In a composition of the invention, the SLPs are (essentially) constituted of biocompatible and biodegradable material.

Said biocompatible phospholipids and said biocompatible lipidic compound(s) are two physiologically well-tolerated components, and present some interesting characteristics for the delivery of drugs (or of said active compound(s)) by the pulmonary route.

The SLPs are (essentially) composed of physiological compounds present in the endogenous lung surfactant, and are thus less affected by the alveolar macrophages clearance mechanism.

For example, the phospholipids of the SLPs can be a mixture of disaturated phosphatidylcholines, which correspond to an estimated 55% to 80% of phosphatidylcholine (or 45% to 65% of total phospholipids) of the naturally occurring pulmonary surfactant pool.

The endogenous lung surfactant is a complex mixture of lipids and proteins comprising about 85% to about 90% phospholipids (of which about 90% are phosphatidylcholine and 8-10% are phosphatidylglycerol), 6-8% biologically active proteins (Surfactant Proteins, SP-A, SP-B, SP-C and SP-D) and 4-7% neutral lipid (primarily cholesterol) by weight.

It is also interesting to note that the phospholipids present in the lung surfactant are largely saturated, with dipalmitoyl phosphatidylcholine (DPPC), representing up to 40% of the total phospholipids present.

The endogenous lung surfactant is synthesized, processed, packaged, secreted and recycled by type II pneumocytes. It is stored in characteristic lamellar body organelles in the cytoplasm prior to secretion into the alveolar hypophase.

After performing its physical function, the great majority of lung surfactant is reutilised directly or indirectly to augment cellular surfactant stores rather than being lost from the alveolar compartment. Only about 10% to about 15% of alveolar surfactant appears to be taken up into macrophages. Most of this surfactant is presumably degraded rather than reutilised, and this pathway probably accounts for much of the loss from the alveolar compartment over time.

A small fraction of 2-5% of alveolar surfactant is also thought to be cleared to the airways.

The recycling of alveolar surfactant phospholipids and apoproteins within the type II cell involve that some surfactant components are transported to the lamellar bodies without degradation and are combined intact with newly synthesized surfactant, while others are catabolized to products that are incorporated into synthesis pathways.

Recycling of phospholipids, proteins, and other components present in exogenous surfactants by type II pneumocytes is known to occur.

In other words, the exogenous phospholipids (from the SLPs or active SLPs of the invention) are expected to be recycled, i.e. reutilised in the endogenous surfactant pool, and thus are expected to be well tolerated.

Advantageously, in a composition according to the invention, each particle comprises or consists of:
- one, two, three, four or more biocompatible phospholipids selected from the phospholipid classes including anionic phospholipids, cationic phospholipids, zwitterionic phospholipids and neutral phospholipids, such as for example phosphatidylcholine, phosphatidyl glycerol, phosphatidyl-ethanolamine, phosphatidyl-inositol, phosphatidyl-serine, and
- one, two, three, four or more biocompatible lipidic compounds, which are not phospholipids, such as glycerol esters (e.g. mono-, di- or tri-glycerides, in particular glycerol monostearate, glycerol behenate), fatty alcohols (in particular with 16 C or more), fatty acids (in particular with 16 C or more), ethers of fatty alcohols, esters of fatty acids, hydrogenated oils, polyoxyethylenated derivatives, sterols (e.g. cholesterol and its derivatives, in particular cholesterol esters) or any derivatives thereof, and
- optionally, one, two, three, four or more active compounds.

Preferably, in a composition according to the invention, each particle comprises or consists of :
- one, two three four or more saturated biocompatible phospholipids selected from the class of phosphatidylcholine having a high transition temperature such as dipalmitoyl phosphatidylcholine (DPPC), distearoyl phosphatidylcholine (DSPS), dibehenyl phosphatidylcholine (DBPC), palmitoyl-stearoyl phosphatidylcholine (PSPC) palmitoyl-behenyl phosphatidylcholine (PBPC), stearoyl-behenyl phosphatidylcholine (SBPC), saturated phospholipids with longer fatty acid residues or any derivatives thereof,
- one, two, three, four or more biocompatible lipidic compounds with high transition temperature, which are not phospholipids, such as glycerol esters (e.g. mono-, di- or tri-glycerides, in particular glycerol monostearate, glycerol behenate), fatty alcohols (preferably cetyl alcohol, steary alcohol, cetostearyl alcohol or fatty alcohols with more carbon atoms), fatty acids (preferably palmitic acid, stearic acid, behenic acid or fatty acids with more carbon atoms), ethers of fatty alcohols, esters of fatty acids, hydrogenated oils, polyoxyethylenated derivatives, sterols (e.g. cholesterol and its derivatives, in particular cholesterol esters) or any derivatives thereof, and
- optionally, one, two, three, four or more (micronized) active compounds.

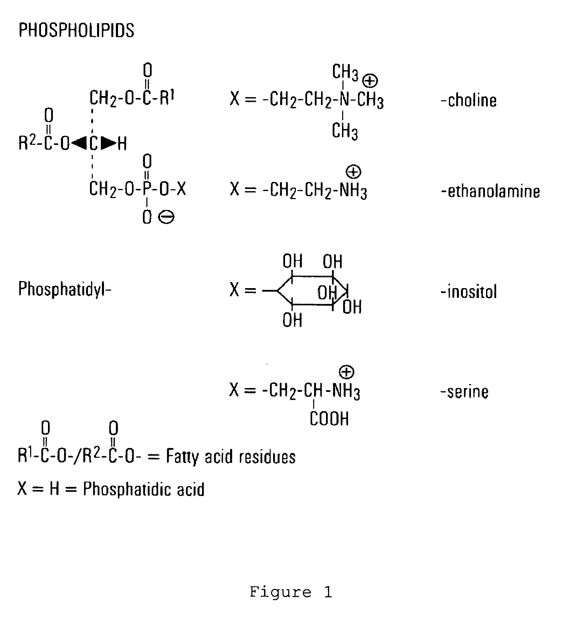

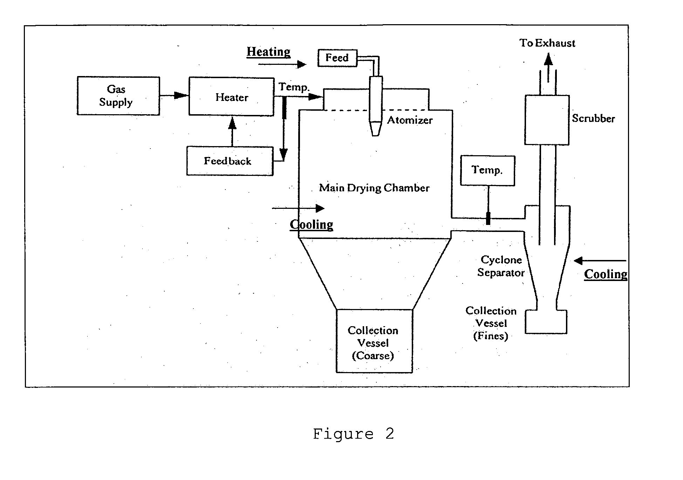

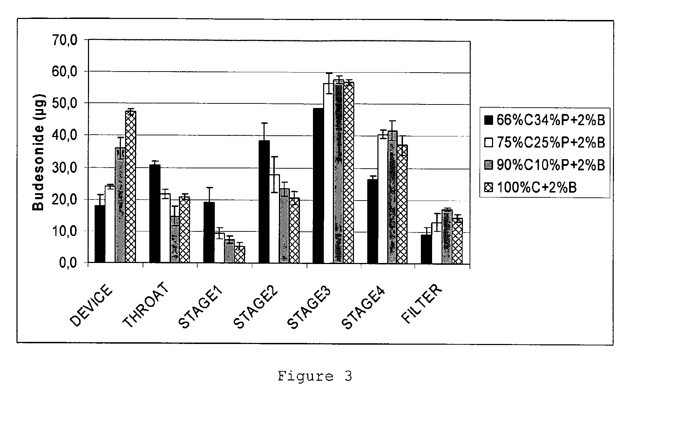

The phospholipids that can be used in a composition of the invention can have a structural formula as given in figure 1, wherein R¹ and R² are fatty acid residues, and wherein R¹ and R² can be the same or can be different.

Preferably, phospholipids to be used in a composition of the invention have a high phase transition temperature (T_{c}) (also referred to as the melting temperature) higher than about 35°C or 40°C, more preferably higher than about 45°C, 46°C, 47°C, 48°C or 49°C, and even more preferably higher than 50°C, 51°C, 52°C or 53°C.

Preferred biocompatible phospholipids of the invention are purified and saturated phosphatidylcholine (e.g. more than about 85 wt.%, preferably more than about 90 wt.% or more than about 95 wt.% in the final purified product), in particular a combination of distearyl-phosphatidylcholine (DSPC) and dipalmitylphosphatidylcholine (DPPC) .

Examples of biocompatible phospholipids with a high phase transition temperature (T_{c}) are Phospholipon® 90H, Phospholipon® 100H (Nattermann Phospholipid GmbH, Köln, Germany), comprising respectively 90% and 95% of hydrogenated phosphatidylcholine, consisting of 85% distearyl-phosphatidylcholine (DSPC) and 15% dipalmitylphosphatidylcholine (DPPC), with a transition temperature Tc of about 54°C.

Similar commercially available high transition temperature phospholipids developed by Lipoid (Ludwigshafen, Germany) are Lipoid® S PC-3 (high purity soy bean saturated phospholipids, comparable to Phospholipon® 100 H), and high purity synthetic phospholipids (Lipoid® PC 16:0/16:0 (DPPC) and Lipoid® PC 18:0/18:0 (DSPC)

For obtaining the products of Nattermann Phospholipid GmbH, crude soy bean lecithin, containing crude phospholipids mixtures and a variety of other compounds such as fatty acids, triglycerides, sterols, carbohydrates and glycolipids, goes through a purification process, without acetone extraction, for preparing very highly purified phospholipids.

An initial ethanolic extraction and column chromatography on silica gel yields lecithin that contains 75% to 85% phosphatidylcholine (Phospholipon® 80). Further chromatography yields lecithin containing over 90% of phosphatidylcholine. A further purification step can be performed. Thus a hydrogenation step generates fully saturated phospholipids (Phospholipon® 90H and 100H).

The phospholipids can play an important role in the physiological tolerance of the inhaled particles.

More over, acting as tension-active ingredient, they can promote the dispersion and dissolution of the inhaled particles in the physiological aqueous fluids.

Active SLP compositions containing large amounts of phospholipids thus can increase the release and the absorption of drugs, especially when the active substances have limited solubility or absorption characteristics.

The more hydrophobic lipids (the additional biocompatible lipidic compound(s)) can act as barrier between aqueous fluids and the active substances, especially for matrix (homogeneous mixture of lipidic and active ingredients in each particle) and encapsulated (micronized active particles coated with lipidic ingredients) active SLP compositions, thereby reducing the rate of absorption of the active substance in the body.

When the proportion by weight of the additional biocompatible lipidic compound(s) largely exceeds that of phospholipids in the active SLPs, the release of the active substance may occur over longer periods than for a composition comprising phospholipids in majority.

Any delayed release of the active substance may provide a lower initial peak of concentration of the active substance, which may result in reduced side effect associated with the active substance.

Therefore, depending on the active ingredient to be used, and on the effect on release sought, the proportion by weight of the hydrophobic lipids (the additional biocompatible lipidic compound(s)) may exceed or not that of the phospholipids in a composition of the invention.

Thus, in each particle of a composition of the invention, the biocompatible phospholipids and the additional biocompatible lipidic compound(s) can be in any weight ratios (zero excepted).

For a SLP composition, wherein each particle consists of biocompatible phospholipids and additional biocompatible lipidic compound(s), said phospholipids can be comprised between about 0,1 wt.% and about 99,9 wt.%, said additional biocompatible lipidic compound(s), constituting the balance, can be comprised between about 0,1 wt.% and about 99,9 wt.%.

For compositions with an expected promoting effect on release and/or on absorption of drugs, and depending on the active compound(s) to be used, the phospholipids can be comprised between about 10 wt.% and about 99,9 wt.%, preferably between about 20 wt.% and about 90 wt.%, more preferably between about 25 wt.% and about 80 wt.%, the additional biocompatible lipidic compound(s) constituting the balance.

For compositions with an expected delay effect on release of the active ingredient(s), the phospholipids can be comprised between about 0,1 wt.% and about 40 wt.%, preferably between about 0,1 wt.% and about 30 wt.%, more preferably between about 5 wt.% and about 20 wt.%, the additional biocompatible lipidic compound(s) constituting the balance.

Generally, the dispersal of the SLPs and/or the dispersal of the micronized drug when added to the SLPs, is promoted when the phospholipids is comprised between about 0,1 wt.% and about 35 wt.%, the additional biocompatible lipidic compound(s) constituting the balance.

For an active SLP composition, wherein each particle consists of biocompatible phospholipids, at least one additional biocompatible lipidic compound and at least one active compound, the weight ratio biocompatible phospholipids / additional biocompatible lipidic compound(s) can be comprised between about 0,1:99,9 and about 99,9:0,1.

For composition with an expected promoting effect on release and/or on absorption of drugs, and depending on the active compound(s) to be used, the weight ratio biocompatible phospholipids / additional biocompatible lipidic compound(s) can be comprised between about 10:90 and about 99,9:0,1, preferably between about 20:80 and about 90:10, more preferably between about 25:75 and about 80:20.

For compositions with an expected delay effect on release of the active ingredient(s), the weight ratio biocompatible phospholipids / additional biocompatible lipidic compound(s) can be comprised between about 0,1:99,9 and about 40:60, preferably between about 0,1:99,9 and about 30:70, more preferably between about 5:95 and about 20:80.

Generally, the dispersal of the active SLPs is promoted when the weight ratio biocompatible phospholipids / additional biocompatible lipidic compound(s) is comprised between about 0,1:99,9 and about 35:65.

Preferred additional biocompatible lipidic compounds to be used in a composition of the invention are cholesterol, cholesterol acetate, and/or glycerol behenate.

Advantageously, in a composition of the invention, each particle comprises or consists of biocompatible phospholipids characterized by a high phase transition temperature (T_{c}) (preferably higher than about 35°C, more preferably higher than about 45°, and even more preferably higher than 50°C) and additional biocompatible lipidic compound(s) selected from the group consisting of cholesterol, cholesterol acetate and glycerol behenate.

Preferably, in a composition of the invention, each particle comprises or consists of biocompatible phospholipids characterized by a high phase transition temperature (T_{c}) (higher than about 35°C or 40°C, more preferably higher than about 45°C, 46°C, 47°C, 48°C or 49°C, and even more preferably higher than 50°C, 51°C, 52°C or 53°C) and cholesterol.

In a preferred composition of the invention, said phospholipids are Phospholipon® 90H and/or Phospholipon® 100H and said additional biocompatible lipidic compound(s) is/are cholesterol, cholesterol acetate and/or glycerol behenate.

In a preferred composition of the invention, each particle comprises or consists of Phospholipon® 90H and/or Phospholipon® 100H, and cholesterol.

Advantageously, the weight ratio Phospholipon® / cholesterol is comprised between about 0,1:99,9 and about 50:50, preferably between about 1:99 and about 40:60, or between about 5:95 and about 35:65, or between about 5:95 and about 30:70, more preferably between about 10:90 and about 30:70, and even more preferably between about 10:90 and about 25:75.

Advantageously the proportion by weight of said additional biocompatible lipidic compound(s) exceeds that of the biocompatible phospholipids in a composition of the invention.

Preferably, said biocompatible phospholipids (e.g. Phospholipon®) and said additional biocompatible lipidic compound(s) (e.g. cholesterol, cholesterol acetate, glycerol behenate) are respectively present in weight ratios of from about 0,1:99,9 to about 40:60, preferably of from about 1:99 to about 40:60, or of from about 5:95 to about 35:65, or of from about 5:95 to about 30:70, more preferably of from about 10:90 to about 30:70, and even more preferably of from about 10:90 to about 25:75.

In a composition of the invention, the active compound(s) can be any drug(s) which are usually administered nasally or orally, in particular by inhalation, e.g. for the treatment of respiratory diseases.

The active compound(s) can also be any drug(s) that can be administered nasally or orally by inhalation in order to reach the systemic circulation.

The active compound(s) can be anti-histaminic or anti-allergic agents, steroids (for example one or more compound selected from the group consisting of beclomethasone dipropionate, budesonide, flucatisone, and any physiologically acceptable derivatives), β-agonists (for example one or more compound selected from the group consisting of terbutaline, salbutamol, salmoterol, formoterol, and any physiologically acceptable derivatives), anti-cholinergic agents (for example one or more compound selected from the group consisting of ipatropium, oxitropium, tiotropium, and any physiologically acceptable derivatives), cromones (for example sodium cromoglycate or nedocromil), leukotriene antagonist receptors.

The active substances can also be antibiotics or any antimicrobial agents, antiviral agents, antipain agents, anticancer agents, muscle relaxants, antidepressants, antiepileptics, hypotensives, sedatives, or any agents to be used for local delivery of vaccines to the respiratory tract.

The active substances can also be therapeutically active agents for systemic use provided that the agents are capable of being absorbed into the circulatory system via the lung.

The active substances can also be peptides or polypeptides such as DNase, leukotrienes, insulin, cyclosporine, interleukins, cytokines, anti-cytokines and cytokine receptors, vaccines, leuprolide and related analogues, interferons, growth hormones, desmopressin, immiunoglobulins, erythropoietin, calcitonin and parathyroid hormone, etc.

In a composition of the invention, the biocompatible phospholipids and the additional biocompatible lipidic compound(s) can be regarded as carriers or fillers.

Advantageously, in a composition of the invention, the weight ratio carriers or fillers / active ingredient(s) is comprised between about 0.01 and about 5000, preferably between about 5 and about 100, more preferably between about 10 and about 50.

Advantageously, the particles in a composition of the invention have a mean particle size smaller than about 100 µm, preferably smaller than about 50 µm, 30 µm, 20 µm and more preferably smaller than about 10µm, 5 µm, or even smaller than about 2 µm, or 1 µm.

The size of the particles may be evaluated by using laser diffraction or any other standard methods of particle sizing or by sizing methods allowing the determination of the aerodynamic diameter of particles according to the methods described in the European or US Pharmacopeas.

A SLP composition of the invention can be used as carrier for obtaining a new composition/formulation comprising or consisting of SLPs and at least one active ingredient, wherein said active ingredient(s) is/are in the form of solid particles, in particular in the form of micronized particles.

Advantageously, in a new formulation of the invention, the active ingredient(s) represent(s) less than about 50 wt.% of said formulation, preferably less than about 20 wt.%, less than about 10 wt.%, or less than about 5 wt.%, and even less than about 3 wt.%, less than about 2 wt.%, or less than about 1 wt.%.

When the use of micronized drug (or active ingredient(s)) is required, the micronized drug particles might have a mean particle size lower than about 20 µm, preferably lower than about 5 µm, such as about 2 µm or about 3 µm.

For example, at least 99% by weight of active particles can have a size lower than 5 µm.

Advantageously, all the SLPs have a mean particle size between about 0,2 µm and about 200 µm, preferably in the range of about 0,2 µm to about 80 µm and more preferably in the range of about 0,5 µm to about 20 µm.

Blends, in different proportions, of SLPs having larger particle size (e.g. diameter of about 60 µm, 80 µm, 100 µm, 150 µm or more) and SLPs having smaller particle size (e.g. diameter of less than about 60 µm, 50 µm, 20 µm, 10 µm, 5 µm, 2 µm, 1 µm, 0,5 µm or even less) can be considered in order to enhance the flowability of the compositions of the invention and to promote the delivery of relatively large proportion of active compounds into the lung.

Advantageously, the particles obtained according to the invention are spherical with a smooth surface and are present as loose agglomerates with important dispersal properties during inhalation.

The new formulation according to the invention can be used in dry powder inhalers. Said dry powder inhaler can be for example a multidose system (reservoir system) or a monodose system, in which the powder is pre-packaged in either capsules (hard gelatine, hydroxypropylmethylcellulose (HPMC), or other pharmaceutically acceptable capsules) or in blisters.

A method for making a composition according to the invention is provided, comprising the steps of :
- preparing a solution or a suspension (or colloidal dispersion) containing biocompatible phospholipids, at least one additional biocompatible lipidic compound, and optionally at least one active compound,
- converting said solution or suspension into particles, and
- optionally adding at least one active compound in particulate form.

For making a SLP composition of the invention, the method comprises the steps of :
- preparing a solution or suspension comprising or consisting (essentially) of biocompatible phospholipids and at least one additional biocompatible lipidic compound, and
- converting said solution or suspension into particles.

For making an active SLP composition of the invention, the method comprises the steps of :
- preparing a solution or a suspension comprising or consisting (essentially) of biocompatible phospholipids, at least one additional biocompatible lipidic compound and at least one active compound, and
- converting said solution or suspension into particles.

In the case of suspensions, some components of the formulation might be partially or totally at the solute state.

Advantageously, in a method of the invention, the biocompatible phospholipids, which may have a formula as given in figure 1, wherein R¹ and R² (equal or different) are fatty acid residues, show a high phase transition temperature (Tc), preferably higher than about 35°C or 40°C, more preferably higher than about 45°C, 46°C, 47°C, 48°C or 49°C, and even more preferably higher than 50°C, 51°C, 52°C or 53°C.

Advantageously, in a method of the invention, the additional biocompatible lipidic compound(s) is/are selected from the group consisting of glycerol esters (e.g. mono-, di-, and tri-glycerides, in particular glycerol monostearate, glycerol behenate), fatty alcohols (preferably with 16, 18 or more carbon atoms), fatty acids (preferably with 16, 18 or more carbon atoms), sterols (e.g. cholesterol, cholesterol esters), and any derivatives thereof.

In a preferred method of the invention, said phospholipids are purified and saturated phosphatidylcholine, e.g. Phospholipon® 90H and/or Phospholipon® 100H, said additional biocompatible lipidic compound(s) is/are cholesterol, cholesterol acetate and/or glycerol behenate.

In a method according to the invention, for preparing said solution / suspension, an appropriate solvent system is chosen on the basis of the solubility of the different compounds.

Water or any aqueous solution, ethanol, isopropanol and methylene chloride are examples of suitable solvent systems that can be used in a method of the invention.

The solvent system used can be heated in order to allow the dissolution of ingredient showing limited solubility characteristics.

Advantageously, the solvent system used is heated up to about 60°C, about 65°C, or about 70°C maximum.

When the lipidic ingredients and, if present, the active substance(s), are not soluble in the solvent system chosen, a method for making a composition of the invention may further comprise, after the step of preparing a suspension containing said phospholipids, said additional biocompatible lipidic compound(s) and optionally said active compound(s), and before the conversion step, a step of homogenizing said suspension.

A preferred process for converting said solution or suspension into particles consists of the spray drying process.

Spray-drying is a one step process that converts a liquid feed (solution, coarse suspension, colloidal dispersion, etc.) to a dried particulate form.

The principal advantages of spray-drying with respect to a composition of the invention are the ability to manipulate and control particle size, size distribution, shape, and density in addition to macroscopic powder properties such as bulk density, flowability, and dispersibility.

In classical spray dryers, the inlet and outlet temperatures are not independently controlled. Typically, the inlet temperature is established at a fixed value and the outlet temperature is determined by such factors as the gas flow rate and temperature, chamber dimensions, and feed flow rate.

For the purpose of this invention, the existing process and device had to be improved for a better drying efficiency and/or to diminish and even prevent (partial) melting or softening of the lipidic components.

On the one hand, the spraying gaz is heated in order to bring the nebulized droplets of the sprayed solution or suspension directly in contact with pre-heated gaz and thus, to increase the evaporation of the solvent system.

The temperature of the spraying gaz might be as high as possible, in accordance with the ebullition temperature of the solvent system used, but might not be too high in order to avoid any excessive softening or melting of lipidic ingredients.

Spraying gaz temperatures of about 60°C, of about 65°C, or of about 70°C can be used in this purpose.

A method of the invention can comprise a further step of heating the solution or suspension prepared, before the step of spray drying.

In the (main) drying chamber, after the solution or suspension is converted into particles, said particles are cooled down for example by means of dried cold air brought at the bottom level of said (main) drying chamber (see Figure 2).

Said dried cold air can be brought by means of an air cooling system equipped with an air dryer.

Furthermore, a jacketed cyclone with cold water circulation can be used to cool the cyclone separator walls and thus reduce even more the adhesion of the lipidic particles.

A method for making a composition according to the invention can thus comprise the steps of :
- preparing a solution or a suspension containing biocompatible phospholipids, at least one additional biocompatible lipidic compound, and optionally at least one active compound,
- optionally, heating said solution or suspension to reach a temperature up to about 40°C, up to about 50°C, up to about 60°C, or up to 70°C,
- in case of a suspension, homogenizing said suspension,
- converting the said solution or suspension into particles by feeding a (modified) spray drying system comprising :

- a gaz heating system in order to increase the temperature of the spraying gaz,
- a dried cold air generating system in order to cooling down the spray dried particles,
- a cyclone separator, the walls of which are cooled by any suitable means, in order to collect the dried particles.

A factorial design study has permitted to determine the optimal conditions of spray-drying for the preparation of SLP when ethanolic solutions of lipids were used.

For example, a method for making a (active) SLP composition of the invention can comprise the steps of :
- preparing a solution (at 60 °C) containing biocompatible phospholipids and at least one additional biocompatible lipidic compound, and optionally at least one active compound, wherein the solvent is ethanol,
- feeding a modified spray drying apparatus with said heated solution for its conversion into particles by adopting the following particular conditions : spraying air heated to 55°C, dried cold air at about -5°C brought at the bottom level of the (main) drying chamber, cyclone separator walls cooled by cold water circulation at 5°C.

A spray drying apparatus is also provided comprising :
- a gaz heating system in order to increase the temperature of the spraying gaz,
- a dried cold air generating system in order to cooling down the spray dried particles,
- a cyclone separator, the walls of which are cooled by any suitable means, in order to collect the dried particles.

In a method of the invention, the step of spray-drying can be replaced by any process suitable for making particles out of a solution, a suspension, or a colloidal dispersion. containing said phospholipids, said additional biocompatible lipidic compound(s) and optionally said active compound(s).

Examples of such processes include freeze drying, spray freeze drying, gas phase condensation, or supercritical fluid methods.

When one or more ingredients of the composition are not soluble in the solvent system (i.e. suspension system), milling processes or high speed or high pressure homogenization techniques can be used in order to obtain appropriate particle size of the active or lipidic ingredients prior to the step of conversion of a suspension to dried particles.

In a method of the invention, the SLPs used as carrier and micronized particles of active ingredient may be mixed in any suitable way. The SLPs are preferably sieved (using stainless steel sieves of aperture diameters 315 µm for example) prior to be blended with the micronized active particles in an appropriate mixer.

Three different laboratory scale mixers namely Turbula 2C as a tumbling mixer, Collette MP-20 as a planetary mixer and Mi-Pro as a High shear mixer have shown quite satisfactory powder homogenisation results for mixing time comprised between about 10 minutes and about 60 minutes at optimal speeds.

A composition of the invention, in a powder form, may be used in a dry powder inhaler.

In a composition of the invention, the lipidic ingredients can promote the dispersal of the active particles to form an aerosol on actuation of the inhaler.

A composition of the invention may also comprise any suitable.propellant and/or excipient for use in a pressurized metered dose inhaler (pMDI) and/or nebulizers.

A composition of the invention may also be formulated in suspension of active SLPs in appropriate vehicle as pressurized or ultra sonic nebulizers.

In a composition of the invention, the active substance may exert its pharmacological effect over a significantly longer period than the period over which the active substance exerts it pharmacological effect when inhaled alone.

In a composition of the invention, the absorption of the active ingredient can be promoted after inhalation in comparison with formulation for inhalation containing the active ingredient alone.

In a composition of the invention, the tolerance to the inhaled particles is increased in presence of lipidic ingredients.

A composition of the invention may also contain particles of a common excipient material for inhalation use, as fine excipient particles and/or carrier particles.

A composition of the invention may also contain any acceptable pharmacologically inert material or combination of materials. For example, sugar alcohols; polyols such as sorbitol, mannitol and xylitol, and crystalline sugars, including monosaccharides (glucose, arabinose) and disaccharides (lactose, maltose, saccharose, dextrose); inorganic salts such as sodium chloride and calcium carbonate; organic salts such as sodium lactate; other organic salts such as urea, polyssacharides (starch and its derivatives); oligosaccarides such as cyclodextrins and dextrins.

The invention is described in further details in the following examples, which are intended for illustration purposes only, and should not be construed as limiting the scope of the invention in any way.

### Examples

### Example 1

This example illustrates one aspect of the invention : new formulations based on blends of SLPs, used as pharmaceutical carrier, and micronized active compounds.

Said new formulations comprise, based on the total weight, 98% of SLPs used as carriers (with a weight ratio Phospholipon® 90H / cholesterol ranging from 40:60 to 0.1:99.9) and 2% micronized budesonide.

A method for making the SLP compositions comprises the preparation of a solution of cholesterol, and a solution of Phospholipon 90H. Different solvent systems can be used, preferably ethanol, isopropanol or methylene chloride.

The solutions have to be combined such that the total solute concentration is greater than 1 gram per litre, and spray dried to form SLPs with appropriate particle size for inhalation.

Spray-drying is carried out, using a modified laboratory scale spray dryer, Büchi mini spray dryer B-191 (Büchi Laboratory Techniques, Switzerland).

On the one hand, the spraying gas (air) is heated to increase the drying efficiency, and on the other hand, dried cold air is generated at the bottom level of the main drying chamber, using an air cooling system equipped with an air dryer, in order to decrease the outlet air temperature (see Figure 2).

Furthermore, a jacketed cyclone with cold water circulation is used to cool the cyclone separator walls and thus to reduce the adhesion of the particles.

A factorial design study has permitted to determine the optimal conditions of spray-drying for the preparation of SLPs when ethanolic solutions of lipids were used : inlet air temperature, 70°C; outlet air temperature, 28-34°C; spraying air flow, 800 l/h heated at 55°C; drying air flow, 35m³/h; solution feed rate, 2,5-3.0 g/min; nozzle size, 0,5 mm; generation of cold air -5°C at 10 m³/h; cold water circulation in the jacketed cyclone at 5°C.

Using different weight ratios Phospholipon® 90H / cholesterol, ranging from 40:60 to 0,1:99,9, different SLP compositions are obtained.

For each SLP composition, the particle size distribution is measured by laser diffractometry, using a dry sampling system with a suitable SOP (Standard Operating Procedure), (Scirocco® , Mastersizer 2000, Malvern, UK).

The size distributions are expressed in terms of the mass median diameter d(0,5), i.e. the size in microns which 50% of the sample is smaller and 50% is larger, and in terms of the volume (mass) mean diameter D[4,3].

For obtaining said new formulations, each SLP composition is premixed with active micronized particles of budesonide for between 5 and 15 minutes in a mortar (with a spatula, without crushing), and then blended for between 5 and 30 minutes in a tumbling blender (Turbula Mixer, Switzerland).

Particle size distribution results obtained by laser diffractometry for the different SLP formulations are given in Table 1.

As shown in Table 1, the mass median diameter and the volume mean diameter are tiny and range from 1,7 µm to 3,1 µm and from 2,0 µm to 3,9 µm, respectively.

The particle size distribution results (data not shown) obtained for the different formulations are unimodal, narrow and range from 0,3 µm to 10 µm, with more than 90% of the particles having a diameter below 5,0 µm, which corresponds to upper size limits required for an optimal deep lung deposition.

The addition of micronized budesonide to the SLPs does not affect the particle size distribution's narrowness.

**Table 1 : Particle size distribution of formulations given in example 1, determined by using a laser diffraction method (mean ± s.d. values obtained from 3 determinations, n=3)**

| Formulations (% w/w) | | d(0.5) µm | D[4,3] µm |
|---|---|---|---|
| 66%C34%P* | | 2,9 ± 0,3 | 3,9 ± 0,8 |
| | +2%Bud** | 3,1 ± 0,3 | 3,9 ± 0,9 |
| 75%C25%P | | 1,67 ± 0,03 | 1,88 ± 0,03 |
| | +2%Bud | 1,92 ± 0,03 | 2,25 ± 0,02 |
| 90%C10%P | | 1,60 ± 0,05 | 1,9 ± 0,1 |
| | +2%Bud | 1,70 ± 0,04 | 2,00 ± 0,06 |
| 99.9%C0.1%P | | 1,88 ± 0,04 | 2,4 ± 0,1 |
| | +2%Bud | 2,14 ± 0,03 | 2,80 ± 0,04 |

| | | | |
|---|---|---|---|
| *C : Cholesterol, | | | |
| P : Phospholipon® 90H ; | | | |
| **Bud : Budesonide. | | | |

It can be seen that decreasing the phospholipids / cholesterol ratio (from 34% to 10% of Phospholipon 90H) reduces slightly the mean particle size, whereas SLPs obtained for compositions containing more than 34% of phospholipids tend to stick to the cyclone separator walls of the spray dryer.

This phenomenon can be explained by the physical state of phospholipids during the spray-drying process. Indeed, the phase transition temperature (Tc) of the phospholipids plays an important role in determining the particle size characteristics of the phospholipids-based powders. The higher the phase transition temperature of the phospholipids the lower will be the mean particle size of SLPs and thus, the mass median aerodynamic diameter (MMAD).

In this respect, Phospholipon 90H is preferred, showing one of the highest Tc (around 54°C), for the preparation of SLPs.

For compositions containing more than 34% of Phospholipon 90H, a significant softening of phospholipids during the spray-drying process and consequently a certain aggregation of particles are observed. The use of other phospholipids (saturated phospholipids with longer fatty acids residues) with higher transition temperature should permit to overcome this limitation in the phospholipids content of the compositions.

Beyond 10% Phospholipon 90H, the particles tend to grow slightly.

The Fine Particle Dose (FPD) for the different formulations of SLPs has been determined by the method described in the European Pharmacopoeia 4 for the aerodynamic assessment of fine particle, using Apparatus C - Multi-stage Liquid Impinger (MsLI).

A dry powder inhalation device (Cyclohaler®, Novartis, Switzerland) was equipped with a No. 3 HPMC capsule (Capsugel, France) loaded with 10 mg of the formulations (200 µg of budesonide) so obtained.

In parallel, the In Vitro deposition test has been performed on a marketed form of budesonide (Pulmicort® Turbohaler® 200 µg, Astra Zeneca).

The airflow rate, corresponding to a pressure drop of 4kPa and drawing 4 litres of air through the device, was determined by the uniformity of delivered dose test for each inhaler.

The test was conducted at a flow rate of 100 L/min during 2.4 seconds and at 60 L/min during 4 seconds for the formulations from a Cyclohaler® and the Pulmicort® Turbohaler®, respectively.

At least 3 FPD determinations were performed on each test substance and analysis were carried out by a suitable and validated analytical HPLC method.

The HPLC system consisted of a High-Performance Liquid Chromatography (HP 1100 series, Agilent Technologies, Belgium) equipped with a quaternary pump, an automatically injector, an oven heated at 40°C and a spectrophotometer set at 240 nm. The separation system, as prescribed in the budesonide monograph, (Ph. Eur., 4th. Ed., 2002), was a 12 cm x 4,6 mm stainless steel (5 µm particle size) reversed-phase C18 column (Alltima, Alltech, Belgium). Mobile phase (Acetonitrile-phosphate buffer solution adjusted to pH 3,2 with phosphoric acid, 32:68) was run at a flow rate of 1,5 ml/min.

The mass of test substance deposited on each stage was determined from the HPLC analysis of the recovered solutions. Starting at the filter, a cumulative mass deposition (undersize in percentage) vs. cut-off diameter of the respective stages was derived and the Fine Particle Dose (FPD) was calculated by interpolation the mass of active ingredient less than 5 µm.

The FPD is the dose (expressed in weight/nominal dose) of particles having an aerodynamic diameter inferior to 5µm. It is considered to be directly proportional to the amount of drug able to reach the pulmonary tract in vivo, and consequently, the higher the value of FPD, the higher the estimated lung deposition.

The fine particle assessment results for the formulations and the marketed form of budesonide, represented by the FPD, are summarized in Table 2.

**Table 2 : in vitro deposition study, with formulations given in example 1 vs Pulmicort® Turbohaler® (loaded dose=200µg, n=3)**

| | SLP(66%C 34%P) + 2%Bud | SLP(75%C 25%P) +2%Bud | SLP(90%C 10%P) +2%Bud | SLP(99.9%C 0.1%P) +2%Bud | Pulmicort® |
|---|---|---|---|---|---|
| FPD(µg) | 81 ± 3 | 106 ± 1 | 113 ± 5 | 105 ± 3 | 68 ± 5 |

| | | | | | |
|---|---|---|---|---|---|
| *C : Cholesterol, | | | | | |
| P : Phospholipon® 90H ; | | | | | |
| **Bud : Budesonide. | | | | | |

The different formulations present substantially higher FPD values than the reference, which is very promising.

The results are in accordance with the particle size determination, obtained by laser diffraction, since that the FPD value augments when the formulation content of Phospholipon 90H is reduced from 34% to 10%.

Indeed, as discussed above, the decreasing of Phospholipon content of the formulations tends to reduce the particles aggregation and consequently gives a better deep lung deposition.

On the other hand, it seems that a cholesterol/Phospholipon 90H ratio of 90/10 is the most appropriate one as it gives the highest FPD and the best deposition pattern (Figure 3). -

Surface topographies of these powders were investigated and the scanning electron microphotographs are illustrated in Figure 4.

In the bulk form, Phospholipon 90H appears as aggregated flat pebbles (Fig. 4a, left). The original cholesterol is shown as plate-like fine crystals with diameters of approximately 500-1000 µm (Fig. 4a, right). Processing of the solutions of lipids by spray-drying yielded a powder with a substantially different physical appearance. SEM micrographs of the SLPs show spherical structures consisting of many tiny spherical particles, approximately 0,25-2 µm in diameter, slightly fused and agglomerated (Fig. 4b).

The physical blends of SLP with the active substance appears to consist of these slightly fused and aggregated lipidic micro particles surrounding and interacting with aggregates of flat and irregularly shaped particles of budesonide (Fig. 4c). The tap density is evaluated to be around 0,21 g/cm³.

### Example 2

The FPD of another formulation containing 1% of micronized fluticasone propionate, blended with a SLP composition obtained as described in example 1, wherein the weight ratio Phospholipon® 90H / cholesterol is 10:90, has been determined and compared to a marketed form of drug (Flixotide® Diskus®, GSK).

Particle size distribution results obtained by laser diffractometry for the SLPs are given in Table 3.

They show that the mass median diameter and the volume mean diameter are tiny, 2.7 µm and 3.1 µm respectively.

The particle size distribution result obtained for this formulation is unimodal (data not shown), narrow and range from 0,2 µm to 12 µm, with about 90% of the particles having a diameter below 5 µm, which corresponds to upper size limits required for an optimal deep lung deposition.

The addition of the micronized active substance to the SLPs does not affect the particle size distribution's narrowness.

**Table 3 : Particle size distribution of the SLPs and formulation given in example 2, determined by using a laser diffraction method (n=3).**

| Formulations (% w/w) | | d(0.5) µm | D[4,3] µm |
|---|---|---|---|
| 90%C10%P | | 2,7 ± 0,2 | 3,1 ± 0,2 |
| | +1% Flut** | 2,9 ± 0,4 | 3,4 ± 0,6 |

| | | | |
|---|---|---|---|
| *C : Cholesterol, | | | |
| P : Phospholipon® 90H ; | | | |
| **Flut : Fluticasone propionate. | | | |

The Fine Particle Dose (FPD) has been determined by the method described in the European Pharmacopoeia 4 for the aerodynamic assessment of fine particle, using Apparatus C - Multi-stage Liquid Impinger (MsLI).

A dry powder inhalation device (Cyclohaler®, Novartis, Switzerland) was equipped with a No. 3 HPMC capsule (Capsugel, France) loaded with 10 mg of the formulations (100 µg fluticasone) so obtained.

In parallel, the In Vitro deposition test has been performed on the marketed form of fluticasone propionate (Flixotide® Diskus®, GSK).

The airflow rate, corresponding to a pressure drop of 4kPa and drawing 4 litres of air through the device, was determined by the uniformity of delivered dose test for each inhaler.

The test was conducted at a flow rate of 100 L/min during 2.4 secondes and at 80 L/min during 3 secondes for the formulation from the Cyclohaler® and the Diskus® inhalation device, respectively.

At least 3 FPD determinations were performed on each test substance and analysis were carried out by a suitable and validated analytical HPLC method.

The HPLC system consisted of a High-Performance Liquid Chromatography (HP 1100 series, Agilent Technologies, Belgium) equipped with a quaternary pump, an automatically injector, an oven heated at 30°C and a spectrophotometer set at 240 nm. The separation system was a 12 cm x 4,6 mm stainless steel (5 µm particle size) reversed-phase C18 column (Alltima, Alltech, Belgium). The mobile phase (Acetonitrile - phosphate buffer solution adjusted to pH 3,5 with phosphoric acid - methanol, 15:35:50) was run at a flow rate of 1,5 ml/min.

The mass of test substance deposited on each stage was determined from the HPLC analysis of the recovered solutions. Starting at the filter, a cumulative mass deposition (undersize in percentage) vs. cut-off diameter of the respective stages was derived and the Fine Particle Dose (FPD) was calculated by interpolation the mass of active ingredient less than 5 µm.

The FPD is the dose (expressed in weight for a given nominal dose) of particles having an aerodynamic diameter inferior to 5 µm. It is considered to be directly proportional to the amount of drug able to reach the pulmonary tract in vivo, and consequently, the higher the value of FPD, the higher the estimated lung deposition.

The fine particle assessment results for the formulation and the marketed form of fluticasone propionate, represented by the FPD, are summarized in Table 4.

**Table 4 : In vitro deposition study, with formulation given in example 2 (loaded dose=100µg, n=3) vs. Flixotide Diskus® (loaded dose=500µg, n=3)**

| | SLP(90%C10%P) +1%Flut | Flixotide Diskus® |
|---|---|---|
| FPD(µg) | 35.5 ± 0.4 | 70 ± 10 |
| FPF (%) | 35.5 ± 0.4 | 14 ± 2 |

To compare correctly these data, the FPF should be used (the Fine Particle Fraction in percent, %), i.e. the dose (expressed in weight %) of particles having an aerodynamic diameter inferior to 5 µm in relation to the nominal dose (FPD/loaded dose x100).

The new formulation presents substantially higher FPF value than the reference commercial product (35.5 % versus 14%), which is very promising.

### Example 3

This example illustrate another aspect of the invention : an active SLP composition wherein each particle comprises, by weight, 98% of lipidic fillers and 2% budesonide, with a weight ratio cholesterol / Phospholipon® 90H / budesonide of about 90:08:02).

The method carried out for preparing the active SLP composition comprises the steps of:
- preparing a solution of cholesterol, a solution of Phospholipon® 90H, and a solution of budesonide using an appropriate solvent (ethanol or isopropanol),
- mixing the three solutions such that the total solute concentration is greater than 1 gram per litre, and
- spray drying the resulting solution, using the modified Büchi mini spray dryer B-191 (Büchi laboratory-Techniques, Switzerland) to form particles.

The particle size distribution and the Fine Particle Dose are determined as mentioned in example 1. The results are shown in Table 5.

**Table 5 : Particle size distribution and Fine particle dose of the 90%C08%P02%Bud active SLP composition vs Pulmicort® Turbohaler® (n=3)**

| Formulation | d(0.5) µm | V(4/3) µm | FPD µg |
|---|---|---|---|
| 90%C08%P02%Bud | 1,9± 0,1 | 2,3 ± 0,1 | 108 ± 7 |
| Pulmicort® | - | - | 68 ± 5 |
| Turbohaler® | | | |

| | | | |
|---|---|---|---|
| C : Cholesterol, | | | |
| P : Phospholipon® 90H ; | | | |
| Bud : Budesonide. | | | |

As it can be observed, the mass median diameter and the volume mean diameter are tiny, 1,9 µm and 2,3 µm, respectively. Furthermore, the matrix formulation is found to be greatly superior in term of deposition than the reference.

SEM micrographs show spherical structures consisting of many tiny spherical particles, between approximately 0,25 µm and 2 µm in diameter, slightly fused and agglomerated (Fig. 5a). The tap density is evaluated to be 0,20g/cm³.

The addition of budesonide in order to prepare the lipid matrix form (active SLPs) does not affect the physical appearance of the obtained powder (Fig. 5b).

### Example 4

This example illustrates another embodiment, wherein a SLP composition is prepared with a method comprising the steps of preparing a suspension of phospholipids and cholesterol, homogenizing said suspension and spray drying.

A new formulation is prepared comprising, by weight, 98% of the SLP composition used as carrier (wherein the weight ratio Phospholipon® 90H / cholesterol is of 10:90) and 2% micronized budesonide.

The method for preparing said SLP composition comprises the steps of:
- preparing an aqueous suspension of Phospholipon® 90H and cholesterol,
- homogenizing this suspension with high speed homogenizer at 24000 rpm during 10 minutes,
- pre-milling (7 minutes at 6000Psi then 4 minutes at 12000Psi) and milling for between 5 and 30 minutes at 24000 Psi the aqueous suspension with a high pressure homogeniser,
- spray drying the size reduced suspension, using a modified Büchi mini spray dryer B-191, (Büchi laboratory-Techniques, Switzerland) to form SLPs carriers.

These SLPs are mixed with active micronized particles of budesonide for between 5 and 15 minutes in a glass mortar (with a spatula, without crushing) then blended for between 5 and 30 minutes in a tumbling blender (Turbula Mixer, Switzerland) for obtaining the new formulation.

The particle size distribution and the Fine Particle Dose of this formulation are determined as mentioned in example 1. The results are shown in Table 6.

**Table 6 : Particle size distribution and Fine particle dose of the 90%C10%P + 2%B formulation vs Pulmicort® Turbohaler® (n=3)**

| Formulation | d(0.5) µm | V(4/3) µm | FPD µg |
|---|---|---|---|
| Budesonide (raw material) | 0,8 ± 0,1 | 1,0 ± 0,1 | - |
| 90%C10%P+02%B | 9,9 ± 0,2 | 18,5 ± 0,4 | 84 ± 5 |
| Pulmicort® Turbohaler® | - | - | 68 ± 5 |

| | | | |
|---|---|---|---|
| C : Cholesterol, | | | |
| P : Phospholipon® 90H ; | | | |
| B : budesonide. | | | |

Even if the particles have a diameter above 5,0 µm, there is an optimal deep lung deposition, characterised by an FPD of 84 µg for a loaded dose of 200 µg of budesonide (test conducted at 100 L/min during 2.4 seconds, Cyclohaler® inhalation device).

The higher FDP obtained could be explained by the fact that the small particles of budesonide are easily separated from the lipidic excipients, by the energy of the airflow during the inhalation, and reach the lowest stages of the MsLI apparatus.

### Example 5

This example illustrates another embodiment, wherein an active SLP composition is prepared with a method comprising the steps of preparing a solution of phospholipids cholesterol and budesonide, evaporating the solvent at reduced pressure, and milling the solid residue of evaporation to obtain appropriate particle size for inhalation.

The formulation prepared in this example consists of, by weight, 92% of lipidic fillers and 8% budesonide, with a weight ratio Phospholipon® 90H / cholesterol / budesonide of 60:32:08.

The method carried out for preparing this formulation comprises the steps of:
- preparing a solution of Phospholipon® 90H, cholesterol and budesonide in methylene chloride,
- mixing the three solutions such that the total solute concentration is greater than 1 gram per litre,
- evaporating the solvent slowly in a rotary evaporator at reduced pressure, and
- milling by means of an air jet-mill (MCOne jet-mill, Jetpharma, Italy), to obtain micronized active SLPs.

The particle size distribution and the Fine Particle Dose are determined as mentioned in example 1.

### Example 6

According to example 1, the invention features a composition having particles comprising, by weight, 98% of SLPs used as lipidic carrier (with a weight ratio cholesterol acetate / Phospholipon® 90H 90:10) and 2% micronized budesonide. A solution containing 100 gram per litre of the combined lipids in isopropanol (heated at 55°C), was spray dried to form lipid carrier microparticles with appropriate particle size for inhalation. The SLPs (carrier) are premixed with active micronized particles of budesonide for between 5 and 15 minutes in a mortar (with a spatula, without crushing), and then blended for between 5 and 30 minutes in a tumbling blender.

### Example 7

According to example 1, the invention features a composition having particles comprising, by weight, 98% of SLPs used as lipidic carrier (with a weight ratio glycerol behenate / Phospholipon® 90H 90:10) and 2% micronized budesonide. A solution containing 100 gram per litre of the combined lipid in methylene chloride, was spray dried to form lipid carrier microparticles with appropriate particle size for inhalation. The SLPs (carrier) are premixed with active micronized particles of budesonide for between 5 and 15 minutes in a mortar (with a spatula, without crushing), and then blended for between 5 and 30 minutes in a tumbling blender.

### Example 8 -

According to example 3, the invention features a lipid matrix composition having particles comprising, by weight, 98% of lipidic ingredients as fillers and 2% micronized budesonide, with a weight ratio cholesterol acetate / Phospholipon® 90H / budesonide 90:8:2), wherein the method of preparing the formulation comprises preparing a solution of cholesterol acetate, Phospholipon® 90H and budesonide in isopropanol, and spray drying to form active SLP matrix formulation with appropriate particle size for inhalation.

### Example 9

According to example 3, the invention features a lipid matrix composition having particles comprising, by weight, 98% of lipidic ingredients as fillers and 2% micronized budesonide, with a weight ratio glycerol behenate / Phospholipon® 90H / budesonide 97.9:0.1:2), wherein the method of preparing the formulation comprises preparing a solution of glycerol behenate, Phospholipon® 90H and budesonide in methylene chloride, and spray drying to form active SLP matrix formulation with appropriate particle size for inhalation.

### Example 10:

This example illustrates another embodiment, wherein the formulation is based on blends of fine and coarse SLP, used as pharmaceutical carrier, and micronized active compounds.

Since the micronized drug particles are generally very cohesive and characterized by poor flowing properties, they are usually blended, in dry powder formulations, with coarse particles. It improves particles flowability during filling process and ensures accurate dosing of active ingredients. More over, it is known that a ternary component, constituted of fine particles carrier, can be added in order to reduce the force of adhesion between coarse carrier particles and active particles and give the most effective dry powder aerosol.

The fine spray-dried SLPs (about 2 µm mean diameter) and the coarse SLP (about 80 µm mean diameter), as carriers, are mixed for 10 minutes using a Turbula Mixer 2C tumbling blender. Then the micronized budesonide is added and the ternary blend is mixed for 60 minutes.

### Example 11

This example illustrates another aspect of the invention : a lipid coating composition wherein each particle comprises, by weight, 2% of lipidic ingredients (with a weight ratio Phospholipon® 90H / cholesterol of about 25:75) and 98% of a micronized drug practically insoluble in the coating solution.

The method carried out for preparing this active lipid coating composition comprises the steps of:
- preparing a solution of cholesterol, a solution of Phospholipon® 90H, and a suspension of disodium cromoglycate in ethanol,
- mixing them such that the total solute concentration is greater than 1 gram per litre, and
- spray drying the resulting solution, using the modified Büchi mini spray dryer B-191 (Büchi laboratory-Techniques, Switzerland) to form active lipid coated particles.

## Claims

1. A composition consisting of solid particles, each particle comprising biocompatible phospholipids and at least one additional biocompatible lipidic compound.

2. A composition according to claim 1 wherein the weight ratio of said phospholipids to said biocompatible lipidic compound(s) is comprised between 0,1:99,9 and 40:60.

3. A composition according to claim 1 or 2 wherein said phospholipids have a phase transition temperature higher than 45°C.

4. A composition according to any of claims 1 to 3 wherein said biocompatible lipidic compound(s) is/are selected from the group consisting of cholesterol, cholesterol acetate and glycerol behenate.

5. A composition according to any of claims 1 to 4 wherein said each particle further comprises at least one active compound.

6. A composition according to claim 5 wherein said lipidic compounds and said active compound are homogeneously dispersed in said each particle.

7. A composition according to claim 5 wherein said active compound, in a micronized form, is coated by said lipidic compounds.

8. A composition according to any of claims 1 to 7 further comprising at least one active compound in particulate form.

9. A composition according to any of claims 5 to 8 wherein said active compound(s) is/are selected from the group consisting of :
- anti-histaminic, anti-allergic agents, antibiotics and any antimicrobial agents, antiviral agents, anticancer agents, antidepressants, antiepileptics, antipains,
- steroids, in particular beclomethasone dipropionate, budesonide, flucatisone, and any physiologically acceptable derivatives,
- β-agonists, in particular terbutaline, salbutamol, salmoterol, formoterol, and any physiologically acceptable derivatives,
- anti-cholinergic agents, in particular ipatropium, oxitropium, tiotropium, and any physiologically acceptable derivatives
- cromones, in particular sodium cromoglycate and nedocromil,
- leukotrienes, leukotriene antagonist receptors,
- muscle relaxants, hypotensives, sedatives,
- vaccines,
- (poly)peptides, in particular DNase, insulin, cyclosporine, interleukins, cytokines, anti-cytokines and cytokine receptors, vaccines, leuprolide and related analogues, interferons, growth hormones, desmopressin, immiunoglobulins, erythropoietin, calcitonin and parathyroid hormone.

10. A composition according to any of claims 5 to 8 wherein said active compound is budesonide or fluticasone.

11. A method for making a composition consisting of solid particles, each particle comprising biocompatible phospholipids, at least one additional biocompatible lipidic compound, and optionally at least one active compound, comprising the steps of:
- preparing a solution or a suspension containing said phospholipids, said other biocompatible lipidic compound(s), and optionally said active compound(s),
- converting said solution or suspension into particles.

12. A method for making a composition according to claim 11 wherein the step of converting said solution or suspension into particles is performed by means of a spray drying process.

13. A method for making a composition according to claim 11 or 12 further comprising the steps of :
- optionally, heating said solution or suspension to reach a temperature up to about 60°C or up to about 70°C,
- in case of a suspension, homogenizing said suspension,
- spray drying the said solution or suspension, wherein the spray drying apparatus comprises :
- a gaz heating system in order to increase the temperature of the spraying gaz,
- a dried cold air generating system in order to cool down the spray dried particles, and
- a cyclone separator, the walls of which are cooled by any suitable means, in order to collect the dried particles.

14. Use of a composition according to any of claims 1 to 4 as a carrier of pharmaceutically active compounds.

15. Use of a composition according to any of claims 1 to 10 in a dry powder inhaler.

16. Use of a composition according to any of claims 1 to 10 with a suitable propellant and/or excipient in pressurised metered dose inhalers and/or nebulizers.
